# EUROPEAN PATENT SPECIFICATION

(11) **EP 3 332 774 B1**
(45) Date of publication and mention of the grant of the patent: **29.09.2021**
(21) Application number: 16832513.2
(22) Date of filing: 01.08.2016
(51) Int. Cl.: A61K 9/48

(54) **COMPOSITION FOR SOFT CAPSULE SHELL**
ZUSAMMENSETZUNG FÜR WEICHKAPSELHÜLLE
COMPOSITION POUR UN ENELOPPE DE CAPSULE MOLLE

(30) Priority: 05.08.2015 JP 2015154853; 28.04.2016 JP 2016090647
(43) Date of publication of application: 13.06.2018
(73) Proprietor: Fuji Capsule Co., Ltd., Fujinomiya-shi, Shizuoka 418-0112 (JP)
(72) Inventor: KONDO, Yosuke, Fujinomiya-shi Shizuoka 418-0112 (JP); SANO, Taisuke, Fujinomiya-shi Shizuoka 418-0112 (JP); WATANABE, Kazuhiko, Fujinomiya-shi Shizuoka 418-0112 (JP); SHIMOKAWA, Yoshiyuki, Fujinomiya-shi Shizuoka 418-0112 (JP); SATO, Isao, Fujinomiya-shi Shizuoka 418-0112 (JP)
(74) Representative: Hamer, Christopher K.
(86) International application number: PCT/JP2016/003530
(87) International publication number: WO 2017/022230

(56) References cited:
- EP-A1- 2 108 677
- WO-A1-2014/179831
- WO-A2-2007/143652
- CN-A- 101 711 875
- JP-A- 2013 203 671
- JP-A- 2013 537 902
- JP-A- 2015 007 008
- JP-A- 2015 040 186
- US-A1- 2004 052 839
- US-A1- 2013 302 309

## Description

### Technical Field

The present invention relates to a composition for a soft capsule shell, a soft capsule shell formed from the composition for a soft capsule shell, and a soft capsule formulation having the soft capsule shell filled with a capsule fill. The soft capsule formulation according to the present invention has excellent plasticity and flexibility as well as gloss and transparency.

### Background Art

When the capsule fills to be encapsulated in a soft capsule formulation is a liquid (solution or suspension), plasticity and flexibility are required for a soft capsule shell since it is large in a volume change caused by a temperature change in environment. When the soft capsule shell is poor in plasticity and flexibility, cracks may occur in the soft capsule shell due to the volume change of the capsule fills, resulting in leaking the content. Therefore, a plasticizer is added to the soft capsule shell to enhance its plasticity and flexibility.

As a plasticizer, glycerin is often used. Glycerin is hygroscopic and has thus a moisture-retaining effect, whereas the soft capsule shell containing glycerin softens under conditions of high temperature and high humidity and its surface tends to be adhesive. For this reason, the soft capsule formulation comprising such soft capsule shell has a problem that it adheres to each other or sticks to a storage container during storage.

Sorbitol may also sometimes be used in combination with glycerin as a plasticizer in a shell of a soft capsule formulation, but from the shell of a soft capsule formulation containing sorbitol, sorbitol is known to precipitate as a crystal depending on drying conditions. The soft capsule formulation has a commercial value in terms of its transparent appearance, and therefore, it has been a critical problem that appearance of the soft capsule formulation is impaired by precipitation of its crystal.

In order to solve the problems as described above, a soft capsule shell for a soft capsule formulation without glycerin has been reported (Patent Document 1). However, since glycerin is safe and easy to obtain, it has been necessary to have a soft capsule formulation which is not adherent and has excellent appearance even if glycerin is used as a plasticizer.

On the other hand, a soft capsule shell with a base containing gelatin as a main component and a plasticizer containing sorbitol special (a mixture of sorbitol and sorbitan) and maltitol as main components has been used in a soft capsule formulation for having ibuprofen encapsulated therein (Patent Document 2). For the purpose of obtaining a soft capsule from a non-gelatin, a soft capsule shell which comprises a base consisting of kappa carrageenan (κ-carrageenan), iota carrageenan ( 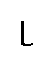 -carrageenan) and sodium starch octenyl succinate, and a plasticizer consisting of Sorbitol Special (a mixture of sorbitol and sorbitan), maltitol syrup and glycerin has been disclosed (Patent Document 3).

The sorbitan has effects of preventing sorbitol from crystallizing and keeping the soft capsule shell transparent. However, sorbitan is an additive that is not included in the list of designated additives, the use of which is approved by the Ministry of Health, Labor and Welfare in Japan, or the existing list of additives. Therefore, it has been desired to obtain transparency of the soft capsule shell without sorbitan.

It is also described that gelatin, glycerin, maltitol and sorbitol are comprised in a soft capsule shell of a soft capsule formulation which is easily chewable and keeps its solubility even after storage for a long period of time (Patent Document 4). However, any soft capsule formulation comprising a soft capsule shell comprising a base consisting of either gelatin or a carrageenan and a starch and a plasticizer consisting of glycerin, maltitol and sorbitol wherein each component in the base and the plasticizer is blended with each other in a specified proportion has not been known to have excellent gloss and transparency.

### Prior Art Documents

### Patent Documents

Patent Document 1: Japanese unexamined Patent Application Publication No. 2013-203671
Patent Document 2: Japanese unexamined Patent Application Publication (Translation of PCT Application) No. 2003-508432
Patent Document 3: Japanese unexamined Patent Application Publication (Translation of PCT Application) No. 2007-524527
Patent Document 4: Chinese Patent No. 101711875

### Summary of the Invention

### Object to be Solved by the Invention

An object of the present invention is to provide a soft capsule formulation in which the soft capsule shell thereof does not easily soften and the surface of the soft capsule shell is resistant to adherence and which has excellent gloss and transparency even if glycerin is used as a plasticizer.

### Means to Solve the Object

According to a first aspect of the invention, there is provided a composition for a soft capsule shell comprising: a base containing a mixture of starch and carrageenan as a main component; a plasticizer containing sorbitol, maltitol and glycerin as main components; and water; wherein: the plasticizer contains 1 to 15 parts by mass of sorbitol, 1 to 30 parts by mass of maltitol and 30 to 60 parts by mass of glycerin, per 100 parts by mass of the mixture of starch and carrageenan.

According to a second aspect of the invention, there is provided a soft capsule shell consisting of a composition of the first aspect of the invention.

According to a third aspect of the invention, there is provided a soft capsule formulation wherein a capsule fill is filled in a soft capsule shell according to the second aspect of the invention.

According to a fourth aspect of the invention, there is provided a method for manufacturing a soft capsule formulation comprising: forming a soft capsule shell by forming a film from a composition for a soft capsule shell comprising a base containing a mixture of starch and carrageenan as a main component and a plasticizer containing sorbitol, maltitol and glycerin as main components, and filling the soft capsule shell with a capsule fill, wherein: the plasticizer contains 1 to 15 parts by mass of sorbitol, 1 to 30 parts by mass of maltitol and 30 to 60 parts by mass of glycerin, per 100 parts by mass of the mixture of starch and carrageenan, wherein the method comprises dispersing in water with stirring each of the base containing a mixture of starch and carrageenan as a main component and the plasticizer, and dissolving them with stirring at 60 to 98°C.

A capsule shell formed from a composition for a soft capsule shell comprising a base containing a mixture of a starch and a carrageenan as a main component and a plasticizer containing sorbitol, maltitol and glycerin as main components wherein each component in the base and the plasticizer is blended with each other in a specified proportion makes it possible to provide a soft capsule formulation which has excellent plasticity and flexibility, neither adheres to each other nor sticks to a storage container, and has excellent gloss and transparency. In addition, the soft capsule formulation according to the present invention can keep its softness even when it contains as a capsule fill a composition which easily hardens over time under extreme conditions.

More specifically, the present invention relates to:
(1) a composition for a soft capsule shell comprising: a base containing a mixture of starch and carrageenan as a main component; and a plasticizer containing sorbitol, maltitol and glycerin as main components, wherein
   the plasticizer contains 1 to 15 parts by mass of sorbitol, 1 to 30 parts by mass of maltitol and 30 to 60 parts by mass of glycerin, per to 100 parts by mass of the mixture of starch and carrageenan;
(2) the composition for a soft capsule shell according to (1), wherein the starch is one or more starches selected from oxidized starch, starch dispersion, moist heat treated starch and acid treated starch;
(3) the composition for a soft capsule shell according to (1) or (2), wherein the carrageenan is *κ-*carrageenan and/or 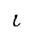 -carrageenan;
(4) a soft capsule shell consisting of the composition for a soft capsule shell according to any one of (1) to (3);
(5) a soft capsule formulation wherein a capsule fill in the soft capsule shell according to (4);
(6) a method for manufacturing a soft capsule formulation comprising: forming a soft capsule shell by forming a film from a composition for a soft capsule shell comprising a base containing a mixture of starch and carrageenan as a main component and a plasticizer containing sorbitol, maltitol and glycerin as main components, and filling the soft capsule shell with a capsule fill, wherein:
   the plasticizer contains 1 to 15 parts by mass of sorbitol, 1 to 30 parts by mass of maltitol and 30 to 60 parts by mass of glycerin, per 100 parts by mass of the mixture of starch and carrageenan;
(7) the method for manufacturing a soft capsule formulation according to (6), wherein the starch is one or more starches selected from oxidized starch, starch dispersion, moist heat treated starch and acid treated starch; and
(8) the method for manufacturing a soft capsule formulation according to (6) or (7), wherein the carrageenan is *κ -*carrageenan and/or 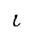 -carrageenan.

The present disclosure also relates to compositions for a soft capsule shell comprising: a base containing gelatin as a main component; a plasticizer containing sorbitol, maltitol and glycerin as main components; and water; wherein: the plasticizer contains 1 to 15 parts by mass of sorbitol, 1 to 30 parts by mass of maltitol and 40 to 60 parts by mass of glycerin, per to 100 parts by mass of the gelatin. The present disclosure also relates to soft capsule shells consisting of said compositions, and soft capsule formulations wherein a capsule fill is filled in said soft capsule shells.

The present disclosure also relates to a method for manufacturing a soft capsule formulation comprising: forming a soft capsule shell by forming a film from a composition for a soft capsule shell comprising a base containing gelatin as a main component and a plasticizer containing sorbitol, maltitol and glycerin as main components, and filling the soft capsule shell with a capsule fill, wherein: the plasticizer contains 1 to 15 parts by mass of sorbitol, 1 to 30 parts by mass of maltitol and 40 to 60 parts by mass of glycerin, per 100 parts by mass of the gelatin, wherein the method comprises dispersing in water with stirring the base containing gelatin as a main component and the plasticizer, and dissolving them with stirring at 60 to 98°C.

### Effect of the Invention

The soft capsule shell according to the present invention obtained from the composition for a soft capsule shell according to the present invention has excellent plasticity and flexibility. Also, the soft capsule formulation according to the present invention formed from the soft capsule shell is transparent so that the content liquid is visible, and the soft capsule formulation has gloss and thus excellent aesthetic appearance and a high commercial value. In addition, the soft capsule formulation according to the present invention has an excellent surface moisture-retaining property and does not have a problem such as cracking.

### Mode of Carrying Out the Invention

### (Composition for soft capsule shell)

The composition for a soft capsule shell according to the present disclosure is not particularly limited, so long as it comprises a base containing either gelatin or a mixture of starch and carrageenan as a main component and a plasticizer containing sorbitol, maltitol and glycerin as main components, wherein when the base is a base containing gelatin as a main component, the plasticizer contains 1 to 15 parts by mass of sorbitol, 1 to 30 parts by mass of maltitol and 40 to 60 parts by mass of glycerin, per 100 parts by mass of the gelatin; and when the base is a base containing a mixture of starch and carrageenan as a main component, the plasticizer contains 1 to 15 parts by mass of sorbitol, 1 to 30 parts by mass of maltitol and 30 to 60 parts by mass of glycerin, per 100 parts by mass of the mixture of starch and carrageenan. The method for manufacturing the soft capsule formulation according to the present disclosure is not particularly limited, so long as it comprises forming a film from either a composition for a soft capsule shell comprising a base containing a mixture of starch and carrageenan as a main component and a plasticizer containing as main components 1 to 15 parts by mass of sorbitol, 1 to 30 parts by mass of maltitol and 30 to 60 parts by mass of glycerin, per 100 parts by mass of the mixture of starch and carrageenan and shaping the formed film into a soft capsule shell, and filling the soft capsule shell with a capsule fill. By the main component used herein is meant a component having a content of 60% by mass or more, preferably 70% by mass or more, and more preferably 80% by mass or more, 90% by mass or more, or 95 to 100% by mass. The composition for a soft capsule shell according to the present invention may further comprise water and any additive in addition to the above-described base and plasticizer. The form of the composition for a soft capsule shell is not particularly limited and the composition may be in the form of solution, suspension, emulsion or paste, or powder, granule or solid, and may be used by dissolving or dispersing it in water or any other liquid raw material. Examples of the additive include a natural pigment, a synthetic pigment, various sweeteners, a preservative, a water activity reducing agent and a pH adjusting agent.

Examples of the gelatin include a gelatin produced by treating a skin, bone, tendon or the like of cows, pigs, chickens, fish or the like with an acid or alkali to obtain a crude collagen and then heating and extracting the obtained crude collagen. As the gelatin, a hydrolyzate or enzyme degradation product of gelatin, and modified gelatin such as succinated gelatin and phthalated gelatin can also be used. Any type of gelatin can be preferably used.

The gel strength of gelatin is preferably 100 to 300 g and more preferably 130 to 250 g. The gel strength can be measured according to JIS K-6503 (2001).

Examples of the starch include potato starch, corn starch, tapioca starch, wheat starch, rice starch, and processed starch such as starches obtained by subjecting these starches to any one of various processing such as physical or chemical processing or the combination of two or more thereof.

Examples of the processed starch obtained by physical processing include pregelatinized starch, moist heat treated starch, and starch dispersion obtained by subjecting starch paste solution to ultrasonic treatment. Examples of the processed starch obtained by chemical processing include etherified starch (such as hydroxypropyl starch and carboxymethyl starch), esterified starch (such as starch acetate, starch octenylsuccinate, phosphated starch and acetylated starch), crosslinked starch (such as phosphate-crosslinked starch and adipate-crosslinked starch), oxidized starch, and acid treated starch.

The oxidized starch is starch obtained by oxidizing starch with an oxidizing agent. Self-modified starch obtained by subjecting starch to thermochemical treatment with an oxidizing agent to reduce its viscosity is also included in the oxidized starch for convenience. Examples of the oxidizing agent include, without being particularly limited to, alkali metal hypochlorite such as sodium hypochlorite; alkaline earth metal hypochlorite such as calcium hypochlorite; alkali metal chlorite such as sodium chlorite and potassium chlorite; and alkaline earth metal chlorite such as barium chlorite.

The carboxyl group content in the oxidized starch is preferably 1.1% by mass or less, particularly preferably 0.8% by mass or less. The carboxy group content in the starch is determined according to the method described in the Ministry of Health, Labor and Welfare Notification No. 485 (October 1, 2008 Official Gazette extra No. 216, pages 28 to 35) (browseable by e-government web page = http://search.e-gov.go.jp/servlet/PcmFileDownload?seqNo=0000035986 [searched on July 14, 2015], "attachment", pages 6-7, (2) "Carboxy group" in the items regarding "purity test").

The acid treated starch is starch obtained by treating starch with an acid. Examples of the acid include inorganic acid or organic acid such as sulfuric acid, hydrochloric acid, phosphoric acid and acetic acid.

The starch dispersion is not particularly limited, so long as it is any starch dispersion obtained by subjecting starch paste solution to ultrasonic treatment. Examples of the starch dispersion include F Smash (manufactured by Futamura Chemical Co., Ltd.) commercially available. The moist heat treated starch is not particularly limited, so long as it is commercially available as "moist heat treated starch", or may be starch obtained by moist heat treatment in the presence of a salt.

The starch used in the present invention is preferably one or more starches selected from oxidized starch, starch dispersion, moist heat treated starch and acid treated starch.

The carrageenan used in the composition for a soft capsule shell according to the present invention is a type of galactan having sulfate group and is known to exist in red algae. Carrageenans can be mainly classified into three types, iota carrageenan ( 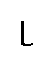 -carrageenan), kappa carrageenan (κ -carrageenan) and lambda carrageenan (λ-carrageenan), depending on their gelation characteristics and structure. Three types of carrageenans, that is, "purified carrageenan", "semirefined carrageenan", "powdered red algae" are prescribed in the provisions of Japanese food additives (see: "Commentaries on List of Items Prescribed in the List of Existing Food Additives", published by the Japan Food Additives Association (1999)), but these differ only in the degree of purification and are essentially all included in carrageenans used in the composition for a soft capsule shell according to the present invention.

In the present invention, 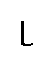 -carrageenan and κ-carrageenan are preferable from the viewpoint of gelling ability. Each of carrageenans may be a pure product or a product containing a standardized substance. Here, the standardized substance is one or more substances selected from a group consisting of sugars such as sucrose, glucose, maltose and lactose, and dextrin. Sucrose and dextrin are preferred. Dextrin is preferably an acid-degraded dextrin or an enzymatically degraded dextrin. The dextrin used herein is a substance different from maltodextrin ("in which degradation has proceeded more than dextrin" (ENCYCLOPAEDIA CHIMICA 8, p. 897, KYORITSU SHUPPAN CO., LTD.)) and cyclic dextrin.

A blended raw material which is obtained by previously mixing 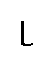 -carrageenan and κ-carrageenan can also be used.

The content of 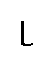 -carrageenan is preferably 10 to 60 parts by mass, more preferably 20 to 50 parts by mass, and particularly preferably 25 to 45 parts by mass, per100 parts by mass of the total mass of all the components of the base, from the viewpoint of strength of a soft capsule shell and fluidity of a solution of a composition for a soft capsule shell.

The content of κ-carrageenan can be appropriately adjusted in the range of 0 to 5.0 parts by mass, per 100 parts by mass of the total mass of all the components of the base, taking into consideration the use of soft capsule formulation and the like.

As described above, the composition for a soft capsule shell according to the present disclosure, which comprises a base containing gelatin as a main component, contains 1 to 15 parts by mass of sorbitol, 1 to 30 parts by mass of maltitol and 40 to 60 parts by mass of glycerin; preferably contains 3 to 15 parts by mass of sorbitol, 3 to 25 parts by mass of maltitol and 40 to 55 parts by mass of glycerin; and more preferably contains 5 to 10 parts by mass of sorbitol, 5 to 15 parts by mass of maltitol and 40 to 50 parts by mass of glycerin, per 100 parts by mass of the gelatin.

As also described above, the composition for a soft capsule shell according to the present invention, which comprises a base containing a mixture of starch and carrageenan as a main component, contains 1 to 15 parts by mass of sorbitol, 1 to 30 parts by mass of maltitol and 30 to 60 parts by mass of glycerin; preferably contains 3 to 15 parts by mass of sorbitol, 3 to 25 parts by mass of maltitol and 35 to 55 parts by mass of glycerin; and more preferably contains 5 to 10 parts by mass of sorbitol, 5 to 15 parts by mass of maltitol and 40 to 50 parts by mass of glycerin, per 100 parts by mass of the mixture of starch and carrageenan.

Sorbitol used as a plasticizer in the composition for a soft capsule shell according to the present invention may be in the form of crystalline powder or sorbitol syrup. Sorbitan is not included in the sorbitol and sorbitol syrup. Maltitol used as a plasticizer in the composition for a soft capsule shell according to the present invention may be in the form of crystalline powder or maltitol syrup. It is also possible to use a liquid mixed raw material, commercially available as "reduced starch syrup", which is a mixture of sorbitol and maltitol, a powder mixed raw material, and a raw material consisting of a eutectic crystal of sorbitol and maltitol.

### (Soft capsule shell)

The soft capsule shell according to the present disclosure, which is a soft capsule shell formed from the composition for soft capsule shell according to the present disclosure, can be manufactured by a conventional method. For example, the soft capsule shell can be prepared by: dispersing in water with stirring each of a base containing either gelatin or a mixture of starch and carrageenan as a main component and a plasticizer containing sorbitol, maltitol and glycerin as main components; dissolving them with stirring at 60 to 98°C; vacuum degassing the resultant; and then spreading it on a plate made of stainless steel or the like with an applicator for uniform thickness. In the case of a punching method with a rotary die type soft capsule filling machine or the like as described below, the soft capsule shell (also referred to as a shell sheet) can be prepared by: dispersing in water with stirring each of a base containing either gelatin or a mixture of starch and carrageenan as a main component and a plasticizer containing sorbitol, maltitol and glycerin as main components; dissolving them with stirring at 60 to 98°C; vacuum degassing the resultant; and then spreading it on a rotary drum at 5 to 60°C with a casting machine.

### (Soft capsule formulation)

The soft capsule formulation according to the present invention is a soft capsule formulation wherein a capsule fill is filled in the soft capsule shell according to the present invention. The soft capsule formulation can be manufactured by a method comprising shaping into the soft capsule shell having a predetermined shape followed by filled it with a capsule fill. However, the soft capsule formulation is preferably manufactured by simultaneously performing shaping into the soft capsule shell having a predetermined shape and filling it with a capsule fill, for example, it can be manufactured by a punching method with a rotary die type soft capsule filling machine or the like, a plating method or the like. Among them, the soft capsule formulation is preferably manufactured with a rotary die type soft capsule filling machine from the viewpoint of industrial productivity.

The method for manufacturing a soft capsule formulation with the rotary die type soft capsule filling machine is a method comprising punching into a capsule shape, from two shell sheets shaped by spreading a composition for a soft capsule shell on a rotary drum, with a pair of rotating dies (die rolls), which method comprises simultaneously performing shaping of the soft capsule and filling with a capsule fill.

The soft capsule formulation according to the present invention can be also obtained as a seamless capsule, which is manufactured by a manufacturing method, referred to as a dropping method or a submerged curing method, utilizing interfacial tension. Specifically, the seamless capsule is manufactured by spraying or dropping a capsule fill from an inner nozzle of a two-fluid nozzle (concentric double nozzle) and a composition for a soft capsule shell according to the present invention from an outer nozzle of the two-fluid nozzle into a gas or a hydrophobic liquid and allowing the composition for shell to be gelled and fixed by cooling or the like.

The shape of the soft capsule formulation is not particularly limited. For example, it is possible to manufacture a soft capsule formulation having an oval (football) type, an oblong (long ellipse) type, round (spherical) type, tubular type and the like, as well as a soft capsule formulation having a special shape such as a self-cut type (also referred to as twist-off type) as illustrated in Japanese Design Registration No. 1492794, Japanese Design Registration No. 1482869, Japanese Design Registration No. 1365845 and Japanese Design Registration No. 1365844, by making a mold for producing each of soft capsule formulation having such shapes.

The soft capsule formulation according to the present invention can find a wide variety of uses such as a pharmaceutical, a quasi-drug, a cosmetic, a food or the like. The composition of the capsule fill is appropriately determined depending on its use. The capsule fill may be in the form of any of solution, suspension, paste, a powder, granule or the like, and is preferably in the form of solution, suspension or paste.

The capsule fills that can be filled in the soft capsule according to the present invention will be illustrated below, but the capsule fill is not limited thereto.

Examples of the fat or oil which can be contained in the capsule fill include avocado oil, almond oil, linseed oil, fennel oil, perilla oil, olive oil, olive squalene, orange oil, orange roughy oil, sesame oil, garlic oil, cocoa butter, pumpkin seed oil, chamomile oil, carrot oil, cucumber oil, beef tallow fatty acid, candlenut oil, cranberry seed oil, brown rice germ oil, rice oil, wheat germ oil, safflower oil, shea butter, liquid shea butter, Japanese basil oil, soybean oil, evening primrose oil, camellia oil, corn oil, rapeseed oil, saw palmetto extract oil, Coix Lacryma-Jobi Ma-yuen seed oil, persic oil, parsley seed oil, castor oil, sunflower oil, grape seed oil, borage oil, macadamia nut oil, meadowfoam seed oil, cottonseed oil, peanut oil, turtle oil, mink oil, egg yolk oil, fish oil, palm oil, palm kernel oil, Japanese wax, coconut oil, long-chain, medium-chain and short-chain fatty acid triglycerides, diacylglyceride, beef tallow, lard, squalene, squalane and pristane, and hydrogenated product of these fats and oils.

Examples of the wax which can be contained in the capsule fill include shellac wax, bees wax, carnauba wax, spermaceti, lanolin, liquid lanolin, reduced lanolin, hard lanolin, cyclic lanolin, lanolin wax, candelilla wax, Japanese wax, montan wax, shellac wax, and rice wax. Examples of the hardened oil which can be contained in the capsule fill include a hardened vegetable oil obtained by hydrogenating a vegetable oil or fat, a hardened oil of beef tallow and a hardened oil of lard.

Examples of lecithin which can be contained in the capsule fill include egg yolk lecithin, soybean lecithin, enzymatically degraded lecithin (lysolecithin), phosphatidylcholine, phosphatidylethanolamine, phosphatidylserine, sphingomyelin, dicetyl phosphate, stearylamine, phosphatidylglycerol, phosphatidic acid, phosphatidylinositolamine, cardiolipin, ceramide phosphorylethanolamine, and ceramide phosphorylglycerol.

The enzymatically degraded lecithin can be obtained by allowing phospholipase A₂ to act, for example, on egg yolk lecithin or soybean lecithin.

Examples of mineral oil which can be contained in the capsule fill include liquid paraffin, vaseline, paraffin, ozokerite, ceresin and microcrystalline wax.

Examples of the fatty acid which can be contained in the capsule fill include natural fatty acid such as lauric acid, myristic acid, palmitic acid, stearic acid, behenic acid, oleic acid, linoleic acid, conjugated linoleic acid, linolenic acid, docosahexaenoic acid, eicosapentaenoic acid, 12-hydroxystearic acid, undecylenic acid, tall oil, and lanolin fatty acid; a synthetic fatty acid such as isononanoic acid, caproic acid, 2-ethylbutanoic acid, isopentanoic acid, 2-methylpentanoic acid, 2-ethylhexanoic acid, and isopentanoic acid; and a fat and oil having any one or more of these fatty acids as its fatty acid composition.

Examples of vitamin which can be contained in the capsule fill include vitamin belonging to a vitamin A group such as retinol, retinal (vitamin A1), dehydroretinal (vitamin A2), carotene and lycopene (provitamin A); vitamin belonging to a vitamin B group such as fursultiamine, thiamine hydrochloride, thiamine sulfate (vitamin B1), riboflavin (vitamin B2), pyridoxine (vitamin B6), cyanocobalamin, methylcobalamin (vitamin B12), folic acids, nicotinic acids, pantothenic acids, biotins, choline and inositols; vitamin belonging to a vitamin C group such as ascorbic acid or its derivatives; a vitamin belonging to a vitamin D group such as ergocalciferol (vitamin D2), cholecalciferol (vitamin D3) and dihydrotachysterol; vitamin belonging to a vitamin E group such as vitamin E or its derivatives, and ubiquinones; vitamin belonging to a vitamin K group such phytonadione (vitamin K1), menaquinone (vitamin K2), menatetrenone, menadione (vitamin K3) and menadiol (vitamin K4); essential fatty acids (vitamin F); carnitine; ferulic acid; γ-oryzanol; orotic acid; vitamin Ps (rutin, eriocitrin and hesperidin); and vitamin U. In addition, lutein can be contained in the capsule fill.

Examples of a stimulant which can be contained in the capsule fill include capsicum tincture, capsicum oil, nonanoic acid vanillylamide, cantharis tincture, ginger tincture, ginger oil, peppermint oil, l-menthol, camphor and benzyl nicotinate.

Examples of a ultraviolet absorbing agent and a ultraviolet screening agent which can be contained in the capsule fill include a benzophenone derivative (such as 2-hydroxy-4-methoxybenzophenone, 2-hydroxy-4-methoxybenzophenone-5-sulfonic acid, sodium 2-hydroxy-4-methoxybenzophenone-5-sulfonate, dihydroxydimethoxybenzophenone, sodium dihydroxydimethoxybenzophenone sulfonate, 2,4-dihydroxybenzophenone, and tetrahydroxybenzophenone), a para-aminobenzoic acid derivative (such as para-aminobenzoic acid, ethyl para-aminobenzoate, glyceryl para-aminobenzoate, amyl para-dimethylaminobenzoate and octyl para-dimethylaminobenzoate), a methoxycinnamic acid derivative (such as ethyl para-methoxycinnamate, isopropyl para-methoxycinnamate, octyl para-methoxycinnamate, 2-ethoxyethyl para-methoxycinnamate, sodium para-methoxycinnamate, potassium para-methoxycinnamate and glyceryl di-para-methoxycinnamate mono-2-ethylhexanoate), a salicylic acid derivative (such as octyl salicylate, phenyl salicylate, homomenthyl salicylate, dipropylene glycol salicylate, ethylene glycol salicylate, myristyl salicylate and methyl salicylate), an anthranilic acid derivative (such as methyl anthranilate), an urocanic acid derivative (such as urocanic acid and ethyl urocanate), a coumarin derivative, an amino acid-based compound, a benzotriazole derivative, a tetrazole derivative, an imidazoline derivative, a pyrimidine derivative, a dioxane derivative, a camphor derivative, a furan derivative, a pyrone derivative, a nucleic acid derivative, an allantoin derivative, a nicotinic acid derivative and a vitamin B6 derivative, and umbelliferone, esculin, benzyl cinnamate, cinoxate, oxybenzone, dioxybenzone, octabenzone, sulisobenzone, benzoresorcinol, arbutin, guaiazulene, shikonin, baicalin, baicalein, berberine, Neo Heriopan, ESCALLOL, zinc oxide, talc and kaolin.

Examples of a whitening agent which can be contained in the capsule fill include a para-aminobenzoic acid derivative, a salicylic acid derivative, an anthranilic acid derivative, a coumarin derivative, an amino acid-based compound, a benzotriazole derivative, a tetrazole derivative, an imidazoline derivative, a pyrimidine derivative, a dioxane derivative, a camphor derivative, a furan derivative, a pyrone derivative, a nucleic acid derivative, an allantoin derivative, a nicotinic acid derivative, vitamin C or its derivatives (such as magnesium ascorbyl phosphate and ascorbyl glucoside), Vitamin E or its derivatives, kojic acid or its derivatives, oxybenzone, benzophenone, arbutin, guaiazulene, shikonin, baicalin, baicalein, berberine, placenta extract, ellagic acid and rucinol.

Examples of a tyrosinase activity inhibitor which can be contained in the capsule fill include vitamin C or its derivatives (such as magnesium ascorbyl phosphate and ascorbyl glucoside), hydroquinone or its derivatives (such as hydroquinone benzyl ether), kojic acid or its derivatives, vitamin E or its derivatives, N-acetyl tyrosine or its derivative, glutathione, hydrogen peroxide, zinc peroxide, placenta extract, ellagic acid, arbutin, rucinol, silk extract, and an extract of a plant (such as chamomile, mulberry, Gardenia jasminoides, Angelica acutiloba, Sanguisorba officinalis, Sophora flavescens, Japanese mugwort, honeysuckle, Phellodendron amurense, Houttuynia cordata, Wolfiporia extensa, Coix Lacryma-Jobi Ma-yuen, Lamium album, Humulus lupulus, Crataegus cuneata, a Eucalyptus, Achillea millefoliuman, althea, Cinnamon Bark, Vitex rotundifolia fruit, Hamamelis virginiana, Morus alba or Morus australis, Isodon japonicus, Platycodon grandiflorum, Cuscuta japonica, Euphorbia lathyris, Iris japonica, Ephedra Herb, Cnidium Rhizome, Aralia Rhizome, Bupleurum Root, Saposhnikovia Root and Rhizome, Glehnia Root and Rhizome, Scutellaria Root, Moutan Bark, Houttuynia Herb, Geranium Herb, Pueraria Root, Glycyrrhiza, Rhus javanica, Aloe arborescens, Cimicifuga Rhizome, safflower, green tea, tea, gambir, blueberry and Astragalus complanatus).

Examples of a melanin pigment-reducing substance and a melanin pigment-decomposing substance which can be contained in the capsule fill include phenylmercuric hexachlorophene, mercuric oxide, mercurous chloride, an aqueous solution of hydrogen peroxide, zinc peroxide, and hydroquinone or its derivatives.

Examples of a turnover promoting agent and a cell activator which can be contained in the capsule fill include hydroquinone, Lactobacillus extract, placenta extract, Ganoderma lucidum extract, vitamin A, vitamin E, allantoin, spleen extract, thymus extract, yeast extract, fermented milk extract, and an extract of a plant (such as Aloe arborescens, Scutellaria Root, Equisetum arvense, Gentiana lutea, Arctium lappa, Lithospermum erythrorhizon, Ginseng, Hamamelis virginiana, Humulus lupulus, Coix Seed, Lamium album, Swertia japonica, Angelica acutiloba, Calendula officinalis, Hydrangea macrophylla var. thunbergii, Hypericum erectum, Cucumis sativus, Thymus vulgaris, Rasmarius officinalis and Petroselium crispum).

Examples of an astringent which can be contained in the capsule fill include succinic acid, allantoin, zinc chloride, zinc sulfate, zinc oxide, calamine, zinc paraphenol sulfonate, aluminum potassium sulfate, resorcinol, ferric chloride and tannic acid (including catechin compounds).

Examples of an active oxygen scavenger which can be contained in the capsule fill include SOD, catalase and glutathione peroxidase.

Examples of an antioxidant which can be contained in the capsule fill include vitamin C or its salts, stearic acid ester, vitamin E or its derivatives, nordihydrogua celetenic acid, butylhydroxytoluene (BHT), butylhydroxyanisole (BHA), hydroxytyrosol, para-hydroxyanisole, propyl gallate, sesamol, sesamolin, gossypol and propolis.

Examples of a lipid peroxide formation inhibitor which can be contained in the capsule fill include β-carotene, and an extract of a plant (a sesame cultured cell, Hydrangea macrophylla var. thunbergii, Hypericum erectum, Hamamelis virginiana, Syzygium aromaticum, melissa, Isodon japonicus, Betula platyphylla var.japonica, Salvia pfficinalis, Rasmarius officinalis, Nandina domestica, a rose fruit, Ginkgo biloba and green tea).

Examples of an anti-inflammatory agent which can be contained in the capsule fill include ichthammol, indomethacin, kaolin, salicylic acid, sodium salicylate, methyl salicylate, acetylsalicylic acid, diphenhydramine hydrochloride, d-camphor, dl-camphor, hydrocortisone, guaiazulene, chamazulene, chlorpheniramine maleate, glycyrrhizic acid or its salts, and glycyrrhetic acid or its salts, Glycyrrhiza extract, Lithospermum erythrorhizon extract, rose fruit extract and propolis.

Examples of an antimicrobial agent, a microbicide and an antiseptic agent which can be contained in the capsule fill include acrinol, sulfur, calcium gluconate, chlorhexidine gluconate, sulfamine, mercurochrome, lactoferrin or its hydrolyzate, an alkyldiaminoethylglycine hydrochloride solution, triclosan, sodium hypochlorite, chloramine T, bleaching flour (calcium hypochlorite), an iodine compound, iodoform, sorbic acid or its salts, propionic acid or its salt, salicylic acid, dehydroacetic acid, para-hydroxybenzoic acid esters, undecylenic acid, thiamine lauryl sulfate, thiamine lauryl nitrate, phenol, cresol, p-chlorophenol, p-chloro-m-xylenol, p-chloro-m-cresol, thymol, phenethyl alcohol, o-phenylphenol, Irgasan CH3565, Halocarban, hexachlorophene, chlorhexidine, ethanol, methanol, isopropyl alcohol, benzyl alcohol, ethylene glycol, propylene glycol, 2-phenoxyethanol, 1,2-pentanediol, zinc pyrithione, chlorobutanol, isopropyl methylphenol, a nonionic surfactant (such as polyoxyethylene lauryl ether, polyoxyethylene nonylphenyl ether and polyoxyethylene octylphenyl ether), an amphoteric surfactant, an anionic surfactant (such as sodium lauryl sulfate and lauroyl sarcosine potassium), a cationic surfactant (cetyltrimethylammonium bromide, benzalkonium chloride, benzethonium chloride and methylrosanilinium chloride), formaldehyde, hexamine, Brilliant Green, Malachite Green, Crystal Violet, Germall, Photosensitizer 101, Photosensitizer 201, Photosensitizer 401, an N-long chain acyl basic amino acid derivative and acid addition salts thereof, zinc oxide, hinokitiol, Sophora flavescens and propolis.

Examples of a moisturizing agent which can be contained in the capsule fill include glycerin, propylene glycol, 1,3-butylene glycol, polyethylene glycol, caprylic/capric triglycerides, glycolic acid (a-hydroxy acid), hyaluronic acid or its salts, chondroitin sulfate or its salts, water-soluble chitin or its derivatives or a chitosan derivative, pyrrolidone carboxylic acid or its salts, sodium lactate, urea, sorbitol, and an amino acid or its derivatives (such as valine, leucine, isoleucine, threonine, methionine, phenylalanine, tryptophan, lysine, glycine, alanine, asparagine, glutamine, serine, cysteine, cystine, tyrosine, proline, hydroxyproline, aspartic acid, glutamic acid, hydroxylysine, arginine, ornithine, histidine, and a sulfate, phosphate, nitrate, citrate or pyrrolidone carboxylate thereof).

Examples of an organic acid which can be contained in the capsule fill include glycolic acid, citric acid, malic acid, tartaric acid, lactic acid, ferulic acid and phytic acid.

Examples of an agent for head hair and head skin which can be contained in the capsule fill include selenium disulfide, an alkyl isoquinolinium bromide solution, zinc pyrithione, biphenamine, thianthol, cantharides tincture, ginger tincture, capsicum tincture, quinine hydrochloride, strong aqueous ammonia, potassium bromate, sodium bromate and thioglycolic acid.

Examples of a fragrance which can be contained in the capsule fill include a natural fragrance including a fragrance of animal origin such as musk, civet, castoreum and ambergris; a fragrance of plant origin such as anise essential oil, angelica essential oil, ylang-ylang essential oil, iris essential oil, fennel essential oil, orange essential oil, cananga essential oil, caraway essential oil, cardamom essential oil, guaiacwood essential oil, cumin essential oil, Kuromoji (Lindera umbellata) essential oil, cassia essential oil, cinnamon essential oil, geranium essential oil, copaiba balsam essential oil, coriander essential oil, Japanese basil essential oil, cider wood essential oil, citronella essential oil, jasmine essential oil, gingergrass essential oil, cedarwood essential oil, spearmint essential oil, peppermint essential oil, star anise essential oil, tuberose essential oil, clove essential oil, orange flower essential oil, wintergreen essential oil, true balsam essential oil, patchouli essential oil, rose essential oil, palmarosa essential oil, hinoki cypress essential oil, hiba essential oil, sandalwood essential oil, petitgrain essential oil, bay essential oil, vetiver essential oil, bergamot essential oil, Peru balsam essential oil, Bois de Rose essential oil, Ho Wood essential oil, mandarin essential oil, Eucalyptus essential oil, lime essential oil, lavender essential oil, linalool essential oil, lemon grass essential oil, lemon essential oil, rosemary (Rasmarius officinalis) essential oil and Japanese peppermint essential oil; and a synthetic fragrance such as coffee flavor and yoghurt flavor.

Furthermore, the capsule fill can contain a nutritional supplement ingredient and/or a health food ingredient. Examples of the nutritional supplement ingredient and the health food ingredient include fish oil, garlic, vitamin B1 and so-called egg oil (a traditional health food material in the form of brown to black liquid which is obtained by cooking egg yolks over gentle heat with stirring using an iron pan or the like for a long time).

The soft capsule formulation according to the present invention is stored and distributed in a packaging form such as a bottling packaging, a PTP packaging and a pouch.

### Examples

Hereinafter, the present invention will be further specifically described with reference to following Examples, but is not intended to be limited thereto.

The raw materials used were as follows:
gelatin (acid-treated gelatin derived from pig skin, manufactured by Nippi,Incorporated);
κ―carrageenan (manufactured by Mitsubishi Shoji Foodtech Co.,Ltd.);
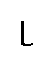 -carrageenan (a product with 40% by mass of a standardized substance (sucrose) added, manufactured by MSC Co., Ltd.);
oxidized starch (Stabirose, manufactured by Matsutani Chemical Industry Co., Ltd.);
starch dispersion (F Smash, manufactured by Futamura Chemical Co., Ltd.);
moist heat treated starch (Soft Starch, manufactured by Sanwa Starch Co., Ltd.);
acid treated starch (Eliane Gel, manufactured by Avebe);
glycerin (manufactured by NOF CORPORATION);
70% D-sorbitol solution (manufactured by Mitsubishi Shoji Foodtech Co.,Ltd.);
maltitol "LESYS" (manufactured by MC-Towa International Sweeteners. Co.,Ltd.); and
reduced starch syrup (manufactured by B Food Science Co., Ltd.).

Examples 1 to 11, Comparative Examples 1 to 5.

Of Examples 1 to 11, Examples 2 to 5 and 7 to 11 are according to the invention, whereas Examples 1 and 6 are reference examples.

Production of soft capsule formulation with rotary die type soft capsule filling machine

### 1. Provision of water-soluble raw materials and the like

An appropriate amount of water (water to be charged) was provided, and glycerin, sorbitol, maltitol and reduced starch syrup in the amount (parts by mass) shown in Table 1 were charged into water and dissolved them in water with stirring.

### 2. Preparation of soft capsule shell

Each of gelatin, κ-carrageenan, 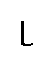 -carrageenan, oxidized starch, starch dispersion and moist heat treated starch in the amount (parts by mass) shown in Table 1 was stirred and dispersed in the liquid obtained in the above 1. Thereafter, the mixture was also dissolved at 60 to 98°C with stirring, vacuum degassed, and then spread on a rotating drum at 5 to 45°C with a casting machine to prepare a sheet-shaped soft capsule shell (also referred to as shell sheet).

### 3. Preparation of blueberry-based capsule fill

Beeswax and glycerin fatty acid ester were added to Japanese basil oil and dissolved them at about 70°C under heating. Then, the heated and dissolved liquid was allowed to cool with stirring to about 30°C to obtain a highly viscous liquid. Blueberry extract powder, lutein suspended oil, dry extract of Astragalus complanatus, beta-carotene suspension and liquid of mixed tocopherols were sequentially added to the above highly viscous liquid, stirred and mixed, and then vacuum degassed. Thereafter, the mixture was subjected to wet grinding with a colloid mill apparatus and sieved as appropriate to obtain a blueberry-based capsule fill.

The blueberry-based capsule fill had a viscosity of 10,000 mPa•s (BII type viscometer, manufactured by Toki Sangyo Co., Ltd.) at 25°C.

### 4. Preparation of soft capsule formulation

Each of the two shell sheets prepared in the above 2 were fed via a lubricating roller and a deflecting roll between a pair of rotating cylindrical dies while filling, between the two shell sheets, a capsule fill selected from vegetable oil (MCT), soybean lecithin and the blueberry-based capsule fill prepared in the above 3, respectively, and subjected to encapsulation while appropriately adjusting the segment temperature depending on the formula of the shells to form soft capsule formulations having an oval (Oval-5) type (football shape). They were stored in a desiccator adjusted to a relative humidity of 20% or less for 24 hours to prepare the soft capsule formulations. Each of the soft capsule formulations in Examples 1 to 11 and Comparative Examples 1, 2, 4 and 5 in which the capsule fill was able to be encapsulated was evaluated for their softness and appearance. The results are shown in Table 2.

### [Evaluation of softness]

Five specialized panelists observed deformation of the soft capsule formulations when pressing them with fingers, and evaluated whether they were soft or hard.

### [Evaluation of appearance]

Five specialized panelists evaluated visually whether or not the soft capsule formulations were beautiful (whether the soft capsule shells were transparent and no ingredient of the soft capsule shells precipitated thereon) and whether or not they were glossy.

**Table 1: Formula of soft capsule shell (parts by mass)**

| | Example 1 | Example 2 | Example 3 | Example 4 | Example 5 | Example 6 | Example 7 | Example 8 | Example 9 | Example 10 | Example 11 | Comparative Example 1 | Comparative Example 2 | Comparative Example 3 | Comparative Example 4 | Comparative Example 5 |
|---|---|---|---|---|---|---|---|---|---|---|---|---|---|---|---|---|
| Gelatin | 100 | 0 | 0 | 0 | 0 | 100 | 0 | 0 | 0 | 0 | 0 | 100 | 0 | 0 | 0 | 0 |
| κ-Carrageenan | 0 | 0.1 | 0.1 | 0.1 | 0.1 | 0 | 0 | 0 | 0 | 0 | 0 | 0 | 0.1 | 0.1 | 0.1 | 0 |
| 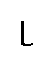 -Carrageenan | 0 | 29.9 | 29.9 | 29.9 | 29.9 | 0 | 30 | 30 | 30 | 30 | 30 | 0 | 39.9 | 29.9 | 29.9 | 30 |
| Oxidized starch | 0 | 30 | 20 | 30 | 30 | 0 | 0 | 30 | 0 | 0 | 0 | 0 | 0 | 20 | 30 | 0 |
| Starch dispersion | 0 | 40 | 0 | 20 | 20 | 0 | 45 | 20 | 0 | 45 | 45 | 0 | 60 | 0 | 20 | 45 |
| Moist heat treated starch | 0 | 0 | 20 | 20 | 20 | 0 | 0 | 20 | 45 | 0 | 0 | 0 | 0 | 20 | 20 | 0 |
| Acid treated starch | 0 | 0 | 0 | 0 | 0 | 0 | 25 | 0 | 25 | 25 | 25 | 0 | 0 | 0 | 0 | 25 |
| Glycerin | 40 | 40 | 50 | 50 | 50 | 40 | 50 | 50 | 50 | 50 | 50 | 45 | 45 | 50 | 50 | 50 |
| 70% D-Sorbitol solution | 10 | 0 | 10 | 10 | 0 | 0 | 0 | 0 | 0 | 5 | 10 | 0 | 0 | 0 | 0 | 20 |
| Maltitol | 10 | 0 | 10 | 10 | 0 | 0 | 0 | 0 | 0 | 5 | 10 | 0 | 0 | 0 | 20 | 0 |
| Reduced starch syrup | 0 | 15 | 0 | 0 | 10 | 20 | 10 | 20 | 30 | 0 | 0 | 0 | 0 | 0 | 0 | 0 |

**Table 2: Formula of content (mg) and its effect**

| | Example 1 | Example 2 | Example 3 | Example 4 | Example 5 | Example 6 | Example 7 | Example 8 | Example 9 | Example 10 | Example 11 | Comparative Example 1 | Comparative Example 2 | Comparative Example 3 | Comparative Example 4 | Comparative Example 5 |
|---|---|---|---|---|---|---|---|---|---|---|---|---|---|---|---|---|
| Blueberry extract powder | 90.0 | 90.0 | 0 | 0 | 0 | 0 | 0 | 0 | 0 | 0 | 0 | 80.4 | 80.4 | 0 | 0 | 0 |
| Lutein suspended oil | 25.0 | 25.0 | 0 | 0 | 0 | 0 | 0 | 0 | 0 | 0 | 0 | 22.3 | 22.3 | 0 | 0 | 0 |
| Dry extract of Astragalus complanatus | 12.5 | 12.5 | 0 | 0 | 0 | 0 | 0 | 0 | 0 | 0 | 0 | 11.2 | 11.2 | 0 | 0 | 0 |
| Beta-carotene suspension | 5.0 | 5.0 | 0 | 0 | 0 | 0 | 0 | 0 | 0 | 0 | 0 | 4.5 | 4.5 | 0 | 0 | 0 |
| Liquid of mixed tocopherols | 35.0 | 35.0 | 0 | 0 | 0 | 0 | 0 | 0 | 0 | 0 | 0 | 31.3 | 31.3 | 0 | 0 | 0 |
| Japanese basil oil | 100.5 | 100.5 | 0 | 0 | 0 | 0 | 0 | 0 | 0 | 0 | 0 | 89.7 | 89.7 | 0 | 0 | 0 |
| Beeswax | 6.0 | 6.0 | 0 | 0 | 0 | 0 | 0 | 0 | 0 | 0 | 0 | 5.4 | 5.4 | 0 | 0 | 0 |
| Glycerin fatty acid ester | 6.0 | 6.0 | 0 | 0 | 0 | 0 | 0 | 0 | 0 | 0 | 0 | 5.4 | 5.4 | 0 | 0 | 0 |
| MCT | 0 | 0 | 0 | 250.0 | 250.0 | 0 | 0 | 0 | 0 | 0 | 0 | 0 | 0 | 0 | 300.0 | 300.0 |
| Soybean lecithin | 0 | 0 | 250.0 | 0 | 0 | 250.0 | 250.0 | 250.0 | 250.0 | 250.0 | 250.0 | 0 | 0 | 250.0 | 0 | 0 |
| Total | 280.0 | 280.0 | 250.0 | 250.0 | 250.0 | 250.0 | 250.0 | 250.0 | 250.0 | 250.0 | 250.0 | 250.2 | 250.2 | 250.0 | 300.0 | 300.0 |
| Formulation (dosage form) | Oval -5 | Oval -5 | Oval -5 | Oval -5 | Oval -5 | Oval -5 | Oval -5 | Oval -5 | Oval -5 | Oval -5 | Oval -5 | Oval -5 | Oval -5 | Formulation impossible | Oval -5 | Oval -5 |
| Softness Appearance | Soft Glossy Beautiful | Soft Glossy Beautiful | Soft Glossy Beautiful | Soft Glossy Beautiful | Soft Glossy Beautiful | Soft Glossy Beautiful | Soft Glossy Beautiful | Soft Glossy Beautiful | Soft Glossy Beautiful | Soft Glossy Beautiful | Soft Glossy Beautiful | Hard Glossy Beautiful | Hard Glossy Beautiful | | Soft Non-glossy | Soft Non-glossy |

As shown in Table 2, the soft capsule formulations in Examples 1 to 11 were soft, glossy and beautiful. The comparisons between Example 1 and Comparative Example 1, and between Example 2 and Comparative Example 2 show that addition of D-sorbitol and maltitol as plasticizers in addition to glycerin achieved softness, gloss and beauty of the soft capsule.

When obtaining the soft capsule formulations filled with soybean lecithin comprising carrageenan and starch as bases, it was a problem that the adhesion portion was weak and only thin adhesion was achieved, but it was possible to fill soybean lecithin in the soft capsule shells according to the present invention (see: Example 3 and Comparative Example 3).

Further, the results of Comparative Examples 4 and 5 show that a combination of glycerin and maltitol and a combination of glycerin and D-sorbitol as a plasticizer gave softness, but it was found that maltitol and D-sorbitol crystallized out and the shells thus became cloudy.

In addition, the soft capsule formulations both after storage at 40°C for 4 months and after cooling storage were subjected to an impact resistance test. The impact resistance test was performed by placing one soft capsule formulation on a station, dropping a weight of 50 g from a height of 10 cm, and counting the number of the soft capsules which were broken among 20 soft capsules (n=20). When the plasticity of the soft capsule shell is lost, the soft capsule is easy to break. Therefore, when the soft capsule formulations exhibit impact resistance, that is, when the number of broken soft capsules is small, the soft capsule shells do not lose plasticity and is excellent in quality. The results of the impact resistance test after storage at 40°C for 4 months are shown in Table 3. The results of the impact resistance test after cooling storage are shown in Table 4.

**Table 3: Impact resistance test after storage at 40°C for 4 months**

| | Example 1 | Modified Comparative Example 1 ^{*1} | Example 2 | Modified Comparative Example 2 ^{*2} |
|---|---|---|---|---|
| At time of production | 0 | 0 | 0 | 0 |
| @40°C, 4 months | 0 | 12 | 0 | 11 |

| | | | | |
|---|---|---|---|---|
| *1 Obtained by modifying the content of glycerin from 45 parts by mass (in Comparative Example 1) to 50 parts by mass. *2 Obtained by modifying the content of glycerin from 45 parts by mass (in Comparative Example 2) to 50 parts by mass. | | | | |

**Table 4: Impact resistance test after cooling storage**

| | | Example 2 | Modified Comparative Example 2 ^{*2} |
|---|---|---|---|
| After cooling storage | @4°C, 7 days | 0 | 11 |
| | @―15°C, 7 days | 0 | 13 |

| | | | |
|---|---|---|---|
| *2 Obtained by modifying the content of glycerin from 45 parts by mass (in Comparative Example 2) to 50 parts by mass. | | | |

It is known that inconveniently, soft capsules containing compositions containing blueberry as capsule fills may easily become hard and may be easily cracked depending on conditions. However, as shown in Table 3, the soft capsules in Examples 1 and 2 had impact resistance and keep softness even after storage at 40°C for 4 months. Table 4 shows that the soft capsule in Example 2 had impact resistance and keep softness even after cooling storage. Therefore, the soft capsule according to the present invention can keep softness and impact resistance even when it contains as a capsule fill a composition which easily hardens over time under extreme conditions, and that it is also very useful in terms of quality improvement.

### Industrial Applicability

The soft capsule shell formed from the composition for a soft capsule shell according to the present invention has excellent plasticity and flexibility. Also, the soft capsule formulation formed from the soft capsule shell according to the present invention is transparent so that the content liquid is visible, and the soft capsule formulation also has gloss and thus excellent aesthetic appearance and a high commercial value. In addition, the soft capsule formulation according to the present invention has an excellent surface moisture-retaining property and does not have a problem such as cracking, and can keep its softness and impact resistance even when it contains as a capsule fill a composition which easily hardens over time under extreme conditions. The soft capsule formulation according to the present invention having a pharmaceutical ingredient, a nutritional supplement ingredient and/or a health food ingredient encapsulated therein can be provided.

## Claims

1. A composition for a soft capsule shell comprising: a base containing a mixture of starch and carrageenan as a main component; a plasticizer containing sorbitol, maltitol and glycerin as main components; and water; wherein:
the plasticizer contains 1 to 15 parts by mass of sorbitol, 1 to 30 parts by mass of maltitol and 30 to 60 parts by mass of glycerin, per 100 parts by mass of the mixture of starch and carrageenan.

2. The composition for a soft capsule shell according to claim 1, wherein the starch is one or more starches selected from oxidized starch, starch dispersion, moist heat treated starch and acid treated starch.

3. The composition for a soft capsule shell according to claim 1 or 2, wherein the carrageenan is κ-carrageenan and/or 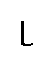 -carrageenan.

4. A soft capsule shell consisting of the composition for a soft capsule shell according to any one of claims 1 to 3.

5. A soft capsule formulation wherein a capsule fill is filled in the soft capsule shell according to claim 4.

6. A method for manufacturing a soft capsule formulation comprising: forming a soft capsule shell by forming a film from a composition for a soft capsule shell comprising a base containing a mixture of starch and carrageenan as a main component and a plasticizer containing sorbitol, maltitol and glycerin as main components, and filling the soft capsule shell with a capsule fill, wherein:
the plasticizer contains 1 to 15 parts by mass of sorbitol, 1 to 30 parts by mass of maltitol and 30 to 60 parts by mass of glycerin, per 100 parts by mass of the mixture of starch and carrageenan, wherein the method comprises dispersing in water with stirring each of the base containing a mixture of starch and carrageenan as a main component and the plasticizer, and dissolving them with stirring at 60 to 98°C.

7. The method for manufacturing a soft capsule formulation according to claim 6, wherein the starch is one or more starches selected from oxidized starch, starch dispersion, moist heat treated starch and acid treated starch.

8. The method for manufacturing a soft capsule formulation according to claim 6 or 7, wherein the carrageenan is κ-carrageenan and/or 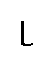 -carrageenan.

## Patentansprüche

1. Zusammensetzung für eine Weichkapselhülle, umfassend: eine Grundlage, enthaltend ein Gemisch aus Stärke und Carrageenan als eine Hauptkomponente; einen Weichmacher, enthaltend Sorbitol, Maltitol und Glycerin als Hauptkomponenten; und Wasser; wobei:
der Weichmacher 1 bis 15 Masseteile Sorbitol, 1 bis 30 Masseteile Maltitol und 30 bis 60 Masseteile Glycerin, pro 100 Masseteile des Gemischs aus Stärke und Carrageenan enthält.

2. Zusammensetzung für eine Weichkapselhülle nach Anspruch 1, wobei die Stärke eine oder mehr Stärke(n) ist, ausgewählt aus oxidierter Stärke, einer Stärkedispersion, feuchter wärmebehandelter Stärke und säurebehandelter Stärke.

3. Zusammensetzung für eine Weichkapselhülle nach Anspruch 1 oder 2, wobei das Carrageenan κ-Carrageenan (kappa-Carrageenan) und/oder 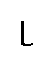 -Carrageenan (iota-Carrageenan) ist.

4. Weichkapselhülle, bestehend aus der Zusammensetzung für eine Weichkapselhülle nach einem der Ansprüche 1 bis 3.

5. Weichkapselformulierung, wobei eine Kapselfüllung in die Weichkapselhülle nach Anspruch 4 gefüllt wird.

6. Verfahren zur Herstellung einer Weichkapselformulierung umfassend: Bilden einer Weichkapselhülle durch Bilden eines Films aus einer Zusammensetzung für eine Weichkapselhülle, umfassend eine Grundlage, enthaltend ein Gemisch aus Stärke und Carrageenan als eine Hauptkomponente und einen Weichmacher, enthaltend Sorbitol, Maltitol und Glycerin als Hauptkomponenten, und Füllen der Weichkapselhülle mit einer Kapselfüllung, wobei:
der Weichmacher 1 bis 15 Masseteile Sorbitol, 1 bis 30 Masseteile Maltitol und 30 bis 60 Masseteile Glycerin, pro 100 Masseteile des Gemischs aus Stärke und Carrageenan enthält, wobei das Verfahren das Dispergieren in Wasser unter Rühren jeweils von der Grundlage, enthaltend ein Gemisch aus Stärke und Carrageenan als eine Hauptkomponente, und dem Weichmacher, und ihr Auflösen unter Rühren bei 60 bis 98 °C umfasst.

7. Verfahren zum Herstellen einer Weichkapselformulierung nach Anspruch 6, wobei die Stärke eine oder mehr Stärke(n) ist, ausgewählt aus oxidierter Stärke, einer Stärkedispersion, feuchter wärmebehandelter Stärke und säurebehandelter Stärke.

8. Verfahren zur Herstellung einer Weichkapselformulierung nach Anspruch 6 oder 7, wobei das Carrageenan κ-Carrageenan und/oder 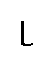 -Carrageenan ist.

## Revendications

1. Composition pour une enveloppe de capsule molle comprenant : une base contenant un mélange d'amidon et de carraghénane comme composant principal ; un plastifiant contenant du sorbitol, du maltitol et de la glycérine comme composants principaux ; et de l'eau ; dans laquelle :
le plastifiant contient 1 à 15 parties en masse de sorbitol, 1 à 30 parties en masse de maltitol et 30 à 60 parties en masse de glycérine, pour 100 parties en masse du mélange d'amidon et de carraghénane.

2. Composition pour une enveloppe de capsule molle selon la revendication 1, dans laquelle l'amidon est un ou plusieurs amidons choisis parmi un amidon oxydé, une dispersion d'amidon, un amidon traité par la chaleur humide et un amidon traité par l'acide.

3. Composition pour une enveloppe de capsule molle selon la revendication 1 ou 2, dans laquelle le carraghénane est un κ-carraghénane et/ou un 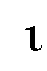 -carraghénane.

4. Enveloppe de capsule molle constituée de la composition pour une enveloppe de capsule molle selon l'une quelconque des revendications 1 à 3.

5. Formulation de capsule molle dans laquelle un contenu de capsule est versé dans l'enveloppe de capsule molle selon la revendication 4.

6. Procédé de fabrication d'une formulation de capsule molle comprenant : la formation d'une enveloppe de capsule molle par formation d'un film à partir d'une composition pour une enveloppe de capsule molle comprenant une base contenant un mélange d'amidon et de carraghénane comme composant principal et un plastifiant contenant du sorbitol, du maltitol et de la glycérine comme composants principaux, et le remplissage de l'enveloppe de capsule molle avec un contenu de capsule, dans lequel :
le plastifiant contient de 1 à 15 parties en masse de sorbitol, 1 à 30 parties en masse de maltitol et 30 à 60 parties en masse de glycérine, pour 100 parties en masse du mélange d'amidon et de carraghénane, dans lequel le procédé comprend la dispersion dans de l'eau avec agitation de chacun de la base contenant un mélange d'amidon et de carraghénane comme composant principal et du plastifiant, et la dissolution de ceux-ci avec agitation à une température de 60 à 98 °C.

7. Procédé destiné à fabriquer une formulation de capsule molle selon la revendication 6, dans lequel l'amidon est un ou plusieurs amidons choisis parmi un amidon oxydé, une dispersion d'amidon, un amidon traité par la chaleur humide et un amidon traité par l'acide.

8. Procédé destiné à fabriquer une formulation de capsule molle selon la revendication 6 ou 7, dans lequel le carraghénane est un κ-carraghénane et/ou un 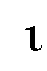 -carraghénane.
